# EUROPEAN PATENT APPLICATION

(11) **EP 4 400 050 A1**
(43) Date of publication of application: **17.07.2024**
(21) Application number: 23386001.4
(22) Date of filing: 12.01.2023
(51) Int. Cl.: A61B 5/268, A61B 5/265, A61B 5/259

(54) **ELECTRODES FOR LONG-TERM RECORDINGS**

(71) Applicant: Cambridge Enterprise, Ltd., Cambridge Cambridgeshire CB2 1TN (GB)
(72) Inventor: The designation of the inventor has not yet been filed
(74) Representative: J A Kemp LLP

(57) **Abstract**

The present invention relates to an electrode comprising a reservoir containing a liquid, and an electrode material in contact with the reservoir and configured to absorb the liquid. The liquid in the reservoir is constituted such that the electrode maintains a low contact impedance with the skin of a subject over a long period of time. The invention also relates to a method of manufacturing an electrode, and a method of continuously hydrating an electrode.

## Description

### Field of the invention

The present invention relates to an electrode, a biosensor, a method of manufacturing an electrode, and a method of continuously hydrating an electrode.

### Background

Measuring electrical activity of organs is useful in a number of medical and research applications.

Wearable electrodes, such as cup electrodes, are used in the recording of electrical activity of organs. For instance, the electrical activity of the brain, heart and muscles may be monitored by electroencephalography (EEG), electrocardiography (ECG) and electromyography (EMG) techniques respectively. Each of these techniques involves placing electrodes on the skin of a patient close to the organ of interest, and measuring and recording over time changes in electric potential detected by the electrodes.

In these applications it is desirable to achieve stable readings over extended periods of time.

Ag/AgCl electrodes are conventionally used as wearable electrodes for electrophysiological sensing applications such as EEG, ECG and EMG. Use of these Ag/AgCl electrodes may involve placing a hydrogel between the electrode material and the skin of the patient so as to maintain adequate contact between the electrode and the skin. Hydrogels are however subject to drying out over time, which causes the electrode-skin contact to deteriorate, thereby decreasing the quality of measurements over time. If an electrode of this sort is to be used for long-term measurements, it is therefore necessary to replenish the supply of hydrogel at the electrode-skin interface at various times during use in order to maintain contact between the electrode and the skin, for instance, by applying the hydrogel directly to the electrode-skin interface by manual means such as a syringe.

There exist Ag/AgCl electrodes having a "gel cavity" to provide the hydrogel to the electrode. A commercial example is the BioSemi FLAT Active electrode, which is an Ag/AgCl electrode comprising a gel cavity intended to reduce motion artifacts and gel dry-out. Another example is the Biopac EL250 Series Reusable Ag-AgCl Electrode, which has an electrolyte gel cavity intended to reduce artifacts due to electrolyte/electrode motion and minimize electrolyte dissipation/drying over long term recordings.

Efforts have also been made to avoid the problem of hydrogel dry-out by producing dry electrodes having adequate adherence to skin during use. Zhang et al have reported the fabrication of dry "PWS electrodes" formed of a film of the conductive polymer PEDOT:PSS blended with the materials WPU and D-sorbitol to improve stretchability and adhesiveness (Zhang, Lei et al. "Fully organic compliant dry electrodes self-adhesive to skin for long-term motion-robust epidermal biopotential monitoring." Nature communications vol. 11, 1 4683. 17 Sep. 2020, doi:10.1038/s41467-020-1 8503-8). Additionally, Yuk et al have reported the fabrication of PEDOT:PSS electrodes by 3D printing (Yuk, H., Lu, B., Lin, S. et al. 3D printing of conducting polymers. Nat Commun 11, 1604 (2020). doi:10.1038/s41467-020-15316-7).

There is a continued need to improve the long-term stability of electrode readings. In particular, there is a need to improve the stability of readings of an electrode in use over periods of, for instance, several days. Additionally, there is a need to devise an efficient and reliable method of manufacturing electrodes that produce stable and high-quality results over long periods of time.

### Summary of the invention

The present invention has been devised in view of the above problems.

A first aspect of the invention is an electrode comprising: a reservoir containing a liquid; and an electrode material in contact with the reservoir and configured to absorb the liquid, wherein the liquid in the reservoir is constituted to maintain an electrode-skin contact impedance of below 100 kΩ at a frequency of about 10 Hz, for a time period of at least 4 days.

By being in contact with the reservoir containing the liquid, the electrode material absorbs said liquid continuously during use. Thus, the absorbent electrode material can maintain a low contact impedance with the skin of a subject ("electrode-skin contact impedance") for a long period of time by absorbing the liquid from the reservoir. Maintaining a low electrode-skin contact impedance in use of an electrode over a long period of time enables the electrode to produce consistent, high-quality and high-accuracy readings with minimal noise over that period. The constitution of the liquid in the reservoir is such that the electrode can exhibit an electrode-skin contact impedance of below 100 kΩ at a frequency of about 10 Hz, for a time period of at least 4 days. This represents a significant improvement over known dry and gel-assisted electrodes, which are subject to degradation in quality of measurements over shorter periods of time, due to a higher electrode-skin impedance or an electrode-skin impedance that tends to increase more rapidly with time.

The time period of at least 4 days may be a continuous time period. The time period of at least 4 days may more specifically be a continuous time period throughout which the electrode is continuously or periodically in contact with the skin of the subject. During the time period of at least 4 days, whenever the electrode is in contact with the skin of the subject the electrode-skin contact impedance is below 100 kΩ at a frequency of about 10 Hz. The beginning of the time period over which the electrode-skin contact impedance is maintained below this value may be the time when the electrode material of the electrode comprising the reservoir containing the liquid becomes exposed. For instance, when the electrode is provided with the liquid in the reservoir and with a seal or cover over the surface of the electrode material that is to be brought into contact with the skin of the subject, the start of the time period may be the moment of removal of the seal or cover from the electrode material. Likewise, when the electrode is provided with the electrode material uncovered but with no liquid in the reservoir, the start of the time period may be the moment of introducing the liquid to the reservoir.

The time period of at least 4 days over which the electrode-skin impedance is maintained below 100 kΩ at a frequency of about 10 Hz may be a time period during which the reservoir is not re-filled or topped up with additional liquid. That is, the electrode-skin contact impedance may be maintained below 100 kΩ for a time period of at least 4 days by virtue of only the liquid that was present in the reservoir at the start of said time period. Re-filling or topping up the reservoir with liquid may be considered to re-set the time period. Similarly, the time period of at least 4 days may be a time period in which no further liquid is applied to the electrode material by other means, such as direct application to the electrode-skin interface. Accordingly, the configuration of the reservoir and the constitution of the liquid within it may be such that the defined electrode-skin impedance is maintained for the defined time period without addition of further liquid during the time period.

The frequency (i.e. AC frequency) of about 10 Hz at which the electrode-skin contact impedance is achieved may be, for example, 10 Hz ± 1 Hz, or exactly 10 Hz. The impedance may be measured by impedance spectroscopy over a range of frequencies of which 10 Hz may be the minimum.

The liquid contained in the reservoir may be an electrolyte. The electrode material in contact with the reservoir may be an absorbent solid material.

Preferably, the time period over which the liquid in the reservoir is constituted to maintain the electrode-skin contact impedance of below 100 kΩ is at least 5 days, preferably at least 10 days, preferably at least 20 days, preferably at least 30 days.

When the liquid is constituted so as to maintain the electrode-skin contact impedance below 100 kΩ for longer periods of time, the quality of readings from the electrode is maintained high for longer.

Preferably, the electrode-skin contact impedance of the electrode which the liquid in the reservoir is constituted to maintain at a frequency of about (e.g. at) 10 Hz for at least 10 days is an electrode-skin contact impedance of below 90 kΩ, preferably below 80 kΩ, preferably below 70 kΩ, preferably below 60 kΩ.

When the liquid is constituted so as to maintain the electrode-skin contact impedance below lower kΩ values for at least 4 days, readings from the electrode are maintained at an even higher quality over the time period.

As regards the kΩ value below which the electrode-skin contact impedance is maintained and the time period over which said contact impedance is maintained, any combination of the kΩ values and time periods disclosed herein are contemplated. In the most preferable example, the liquid is constituted to maintain the electrode-skin contact impedance as low as possible for as long as possible, such as below 60 kΩ for at least 30 days.

In applications where long-term continuous monitoring is needed, it is preferable that the liquid in the reservoir is constituted to maintain the electrode-skin contact impedance of below said kΩ value (e.g. below 100 kΩ), at a frequency of about 10 Hz, for a time period of at least said number of days (e.g. at least 4 days) throughout which the electrode is continuously in contact with the skin of the subject.

That is, the electrode of the present aspect may be configured such that when the electrode is constantly in contact with the skin of the subject over a long, continuous time period, the electrode-skin contact impedance remains low for the entirety of that time period. The continuous time period may be at least 4 days or may preferably be longer (e.g. at least 5 days, at least 10 days, at least 20 days, or at least 30 days). Over that time period the electrode-skin contact impedance may be maintained below 100 kΩ or may preferably be maintained below a smaller value (e.g. maintained below 90 kΩ, maintained below 80 kΩ, maintained below 70 kΩ, or maintained below 60 kΩ). Maintaining a low electrode-skin contact impedance continuously over a time period of continuous use is advantageous in applications that require long-term continuous monitoring of electrical activity in a subject, because the quality of measurements is maintained consistently high throughout the whole time period.

In applications where long-term periodic monitoring is needed, it is preferable that the liquid in the reservoir is constituted to maintain the electrode-skin contact impedance of below said kΩ value (e.g. below 100 kΩ), at a frequency of about 10 Hz, for a time period of at least said number of days (e.g. at least 4 days) throughout which the electrode is periodically in contact with the skin of the subject. The time period and impedance value in this case may be any of those defined herein.

Preferably, the electrode material comprises an electrically conducting polymer. More preferably, the electrode material comprises PEDOT:PSS.

By forming the electrode material of an electrically conducting polymer, the quality of electrode readings in use remains particularly high for particularly long periods of time. PEDOT:PSS is an especially advantageous example of a conducting polymer for use as the electrode material.

The electrode material may comprise conducting metal particles such as silver (Ag) particles.

The electrode material may comprise a natural rubber such as latex.

Preferably, the liquid comprises a deep eutectic solvent or an ionic liquid. Most preferably, the liquid comprises a deep eutectic solvent.

As the liquid contained in the reservoir of the electrode of the present aspect, deep eutectic solvents and ionic liquids are particularly suitable. That is, deep eutectic solvents and ionic liquids have properties making them particularly able to be absorbed into an electrode material to cause the electrode-skin contact impedance of the electrode to remain low for long periods of time. Also, the low tendency of deep eutectic solvents and ionic liquids to dry out over time is beneficial in the manufacture of an electrode.

Deep eutectic solvents are particularly advantageous as the liquid to be contained in the reservoir of the electrode, due to their especially low tendency to dry out over time, their particularly favourable viscosity and tendency to be absorbed into the electrode materials, and their relatively low cost compared with ionic liquids. Thus, a particularly preferable aspect is an electrode as described herein having a deep eutectic solvent as the "liquid" (or as a substantial constituent thereof) that is contained in the reservoir.

Preferably, the electrode further comprises a housing containing the reservoir, the housing being formed of a biocompatible polymer.

The housing may form a rigid or flexible structure to enclose the reservoir on all sides other than that which is in contact with the electrode material. In use of the electrode, the user may pick up and place the electrode in the desired location on the skin of the subject by holding the housing. The housing is formed of a biocompatible polymer, making it suitable for direct contact with the skin of a subject.

Preferably, the housing comprises at least one of: an opening for introducing liquid to the reservoir while the electrode material is in contact with a subject; and an opening to receive a cable.

While the liquid in the reservoir enables the electrode to possess low electrode-skin contact impedance during use for a long time, eventual depletion of the liquid in the reservoir may cause the electrode-skin contact impedance to increase and the quality of measurements to decrease after a period of time. In that case, the presence of a housing having an opening configured to allow the introduction of liquid to the reservoir while the electrode material is in contact with the subject (i.e. during use of the electrode) may enable the reservoir to be re-filled with the liquid without having to dismantle the electrode or even without having to remove the electrode from the skin of the subject. After re-filling the reservoir with the liquid, the electrode may again produce high-quality measurements for a long period of time. The reservoir could in principle be filled an unlimited number of times, such that the capacity of the reservoir places no limit on the length of time over which the electrode may produce high-quality measurements due to having a low electrode-skin contact impedance.

An opening in the housing to receive a cable may facilitate the electrical connection of the electrode material to the power source and/or to an external computer that is used to process measurements from the electrode.

The electrode of the present aspect may comprise multiple distinct layers of electrode material. That is, the electrode material that is in contact with the reservoir may be termed a "first electrode layer", while further layers formed on top of the first electrode layer may be termed "second electrode layer", "third electrode layer" and so on. The distinctiveness of different layers may come about by the nature of the manufacturing process of the electrode as will be described herein. The presence of multiple distinct layers of electrode material can enhance the ability of the electrode to maintain a low electrode-skin contact impedance over a long period of time of continuous or periodic use.

A second aspect of the invention is a biosensor, which comprises the electrode of the first aspect as well as a cable in electrical contact with the electrode material.

The cable may connect the electrode material to a power source and/or to an external computer that is used to process measurements from the electrode. The biosensor may be a wearable biosensor. The biosensor provides all of the advantages of the electrode described herein, and is therefore a useful instrument for use in relatively long-term, continuous or semi-continuous measurement of electrical activity in a subject, such as EEG, ECG and EMG.

A third aspect of the invention is a method of manufacturing an electrode, the method comprising: introducing a liquid to a reservoir, the liquid in the reservoir defining a liquid surface; and depositing an electrode material on the liquid surface, wherein the liquid is constituted such that a surface tension of the liquid supports the electrode material thereon.

The method of the third aspect may be used as a method to manufacture the electrode of the first aspect described above, in which case the "liquid", "reservoir" and "electrode material" of the third aspect may be constituted in any such way as described above in relation to the first aspect, and any additional features of the electrode as described above in relation to the first aspect may be included in the electrode manufactured by the method of the third aspect. Likewise, the electrode manufactured by the method of the third aspect may form the electrode of the biosensor of the second aspect. The electrode produced by the method of the third aspect may thus provide any or all of the advantages discussed herein in relation to the first or second aspect.

In the method of manufacturing an electrode according to the present aspect, the electrode material is deposited on the surface of the liquid in the reservoir, and supported on the liquid by surface tension of the liquid. The liquid may favourably provide a flat and even surface on which a uniform layer of electrode material may be placed, leading to high accuracy and reliability of readings taken by the manufactured electrode during use.

The electrode material may be deposited on the liquid surface in solution. That is, a solution of the electrode material in a solvent (e.g. water) may be deposited onto the liquid surface. For example, when the electrode material is a conducting polymer such as PEDOT:PSS, a solution of the conducting polymer may be deposited on the liquid surface during the manufacture of the electrode. Subsequent drying of the solution of electrode material to remove the solvent may then provide a solid layer of electrode material ("first electrode layer"). The solution of electrode material may be deposited on the liquid surface by drop-casting.

The electrode material, when in the form of a solid layer on the liquid surface (e.g. after drying of a deposited solution of electrode material), may be configured to absorb the liquid that is contained in the reservoir, as described herein in relation to the first aspect.

Preferably, the method of manufacturing an electrode comprises curing the electrode material on the liquid surface to form a first electrode layer. Curing the electrode material on the liquid surface after deposition thereon may enable or accelerate the drying and hardening of the electrode material, forming a first electrode layer suitable to absorb the liquid in the reservoir and to be applied to the skin of a subject. Due to the deposition of the electrode material on the liquid surface prior to curing, the eventual cured first electrode layer has high uniformity and so provides high accuracy and reliability of readings taken by the electrode. When the electrode material is deposited on the liquid surface in solution, such as a solution of PEDOT:PSS, the curing step after the deposition may remove the solvent to leave a dry, uniform layer of the electrode material.

The liquid in the reservoir is preferably constituted such that during any curing steps the liquid does not evaporate significantly. Curing is performed at relatively high temperatures and so can generally cause liquids in the vicinity of the curing to evaporate at an accelerated rate. Evaporation of the liquid that provides the surface on which the electrode material is deposited could lead to imprecision in the formation of the electrode layer (e.g. the first electrode layer) due to disruption in uniformity of the liquid surface on which the electrode material is supported. These imprecisions in formation of the electrode layer may reduce the quality and accuracy of eventual measurements taken by the electrode. By constituting the liquid such that evaporation does not occur to a significant degree, the precision by which the electrode layer is formed can be improved, giving better uniformity and better control of the height of the electrode layer, leading to improved quality and accuracy of measurements taken by the manufactured electrode. For instance, the liquid may be constituted so as to have a vapour pressure of below 1 kPa, more preferably below 500 Pa, more preferably below 200 Pa, more preferably below 100 Pa, more preferably below 50 Pa, more preferably below 30 Pa, taken at a temperature of about 313 K.

Preferably, the liquid is a deep eutectic solvent or ionic liquid. These liquids are especially suitable due to their generally low vapour pressures.

Preferably, the method of manufacturing an electrode further comprises depositing, on the electrode material, an additional amount of the liquid.

That is, after the electrode material has been deposited on the surface formed by the liquid in the reservoir, additional quantities of the same liquid may be deposited on top of the electrode material. This may advantageously improve the speed and uniformity with which the electrode material becomes saturated with the liquid. In the case where the electrode material is deposited on the liquid surface in solution, and subsequently dried (e.g. by curing), depositing an additional amount of the liquid on the electrode material may fill the space left by the evaporated solvent in which the electrode material was dissolved.

The additional amount of the liquid may be deposited by drop-casting. The deposition of the additional amount of the liquid may occur after curing of the electrode material. Additionally or alternatively, the deposition of the additional amount of the liquid may occur during curing of the electrode material. In the latter case, the time needed to manufacture the electrode may be reduced because the time spent curing the electrode material is also used to deposit additional liquid, and stability of the produced electrode may also be enhanced due to the immediate replacement of evaporated solvent and minimisation of time in which the electrode material layer comprises empty spaces.

When the method comprises depositing an additional amount of the liquid on the electrode material, the method may preferably further comprise curing the additional amount of the liquid deposited on the electrode material.

Preferably, the method of manufacturing an electrode comprises forming a plurality of electrode layers including the first electrode layer and a second electrode layer, wherein: the second electrode layer is formed by depositing and curing an additional amount of electrode material on the first electrode layer; and optionally, one or more further electrode layers are formed by sequentially depositing and curing an additional amount of the electrode material on the preceding electrode layer.

That is, the first electrode layer, formed by depositing and drying (e.g. curing) the electrode material on the liquid surface as described herein, may serve as a surface on which to deposit further electrode material to form a second electrode layer. The second electrode layer is formed in a similar way to the first electrode layer, except that the electrode material to form the second electrode layer is deposited on the first electrode layer rather than on the liquid surface on which the first electrode layer was formed. Similarly, a third electrode layer could be formed on the second electrode layer, a fourth electrode layer could be formed on the third electrode layer, and so on. The formation of each layer involves depositing electrode material (e.g. in solution) on the preceding layer, and curing the electrode material to form the solid electrode layer (which may serve as the base for a subsequent layer). The formation of each layer after the first may also incorporate any of the additional steps or features as described herein in relation to the first electrode layer. The formation of multiple layers of electrode material can enhance the ability of the electrode to maintain a low electrode-skin contact impedance over a long period of time of continuous or periodic use.

Preferably, when a plurality of electrode layers are formed, the method further comprises depositing an additional amount of the liquid on the electrode material forming the electrode layers. Said additional amount may be proportional to the amount of electrode layers. For example, after formation of the plurality of electrode layers, an additional amount of the liquid proportional to the number of electrode layers may be deposited onto the top layer. Additionally or alternatively, additional amounts of the liquid may be deposited individually on each electrode layer after its formation, prior to formation of the subsequent electrode layer. The deposition of additional amounts of the liquid can occur preferably while the electrode material on which it is deposited is being cured. The additional liquid may be deposited by drop-casting.

The advantages of depositing an additional amount of the liquid on the electrode material, and of doing so while said electrode material is curing, are discussed herein in relation to the first electrode layer. Those advantages apply also to the formation of a second electrode layer and any subsequent electrode layers. The method may preferably further comprise curing the additional amount of the liquid deposited on the electrode material of the plurality of electrode layers, in which case the length of time of curing and/or the temperature of curing may be proportional to the number of electrode layers formed.

Preferably, the method of manufacturing an electrode further comprises immersing the cured electrode material in an immersion liquid for at least 12 hours, more preferably for at least 24 hours. The immersion liquid may be water. Alternatively, although the immersion liquid is distinct from the "liquid" referred to herein that is introduced to the reservoir to form the electrode, the immersion liquid may nonetheless have the same composition as the aforementioned "liquid" (e.g. a deep eutectic solvent or ionic liquid). Further alternatively, the immersion liquid may be a solution different to the aforementioned "liquid".

Immersing the electrode material in an immersion liquid such as water after curing is an additional treatment step which can enhance the ability of the electrode to maintain a low electrode-skin contact impedance over a long period of time of continuous or periodic use. The step of immersing the electrode material in the immersion liquid occurs after the electrode material has been deposited on the surface of the liquid and cured. When the method includes depositing an additional amount of the liquid on the electrode material, the step of immersing the electrode material in the immersion liquid preferably occurs before the deposition of the additional amount of the liquid on the electrode material. When the method includes forming a plurality of electrode layers, the step of immersing the electrode material in the immersion liquid may occur after the plurality of electrode layers have been formed and cured, and may occur before any (optional) deposition of additional amounts of the liquid on the electrode material.

Preferably, the method of manufacturing an electrode comprises forming a housing by additive manufacturing, the housing containing the reservoir. Most preferably, the housing is formed of a biocompatible polymer.

The housing may form a rigid or flexible structure to enclose the reservoir on all sides other than that which is in contact with the electrode material. In use of the manufactured electrode, the user may pick up and place the electrode in the desired location on the skin of the subject by holding the housing. The housing formed by additive manufacturing may preferably comprise an opening for introducing the liquid to the reservoir while the electrode material is in contact with a subject, and/or an opening to receive a cable. These openings may be advantageous as discussed herein in relation to the first aspect.

Additive manufacturing is an efficient and precise technique for forming the housing. The housing may therefore be formed easily into a shape which facilitates the introduction of liquid and deposition thereon of electrode material to form the electrode. Forming the housing from a biocompatible polymer makes it suitable for direct contact with the skin of a subject.

A fourth aspect of the invention is a method of continuously hydrating an electrode, comprising: bringing an absorbent electrode material of the electrode into continuous contact with a liquid, wherein the liquid in the reservoir is constituted to maintain an electrode-skin contact impedance of below 100 kΩ at a frequency of about 10 Hz, for a time period of at least 4 days.

The electrode continuously hydrated by the method of the fourth aspect may be the electrode of the first aspect, may form part of the biosensor of the second aspect, and may have been manufactured by the method of the third aspect. Any combination of the features or steps described herein in relation to those aspects are also applicable to the fourth aspect.

The electrode is continuously hydrated by bringing it into continuous contact with a liquid, which may be the liquid described herein in relation to the first, second and third aspect and thus provides similar advantages. By continuously hydrating the electrode in this way, the electrode is made able to maintain a low contact impedance with the skin of a subject over a long period of time, leading to consistent, high-quality readings taken from the electrode.

Any of the above aspects may incorporate features described herein in relation to any other of the aspects.

### Brief description of drawings

Figure 1 depicts an electrode according to an embodiment of the invention.
Figure 2 depicts the electrode of figure 1 when in contact with the skin of a subject.
Figure 3 depicts a possible construction of a housing of the electrode, according to an embodiment.
Figure 4 depicts a possible construction of a housing of the electrode, according to an embodiment.
Figure 5 depicts a reservoir containing a liquid prior to deposition thereon of an electrode material, in a method of manufacture of an electrode according to an embodiment.
Figure 6 depicts a multi-layered electrode according to an embodiment.
Figure 7 depicts a possible construction of a housing of the electrode, and deposition thereon of an electrode material.
Figure 8 depicts experimental impedance data comparing an electrode according to an embodiment to a commercially-available electrode over a period of time.
Figure 9 depicts experimental EEG data comparing an electrode according to an embodiment to a commercially-available electrode.
Figure 10 depicts experimental impedance data comparing an electrode according to an embodiment to a commercially-available electrode over a range of frequencies.
Figure 11 depicts experimental impedance data comparing a electrodes of varying amounts of electrode layers according to embodiments to a commercially-available electrode over a range of frequencies.
Figure 12 depicts experimental impedance data of an electrode with silver and latex additives according to an embodiment over a range of frequencies.
Figures 13a and 13b depict a cross-section of an electrode formed with the housing of figure 7.

### Detailed description of embodiments

Exemplary embodiments will hereinafter be described with reference to the drawings.

The invention provides an electrode suitable for use in EEG, ECG, EMG and similar measurement techniques. The electrode of the present invention may be a cup or flat electrode. The electrode is able to provide measurements that are consistently of high quality over long periods, such as several days. By "high quality" it is meant that the measurements taken by the electrode are accurate and exhibit low amounts of noise.

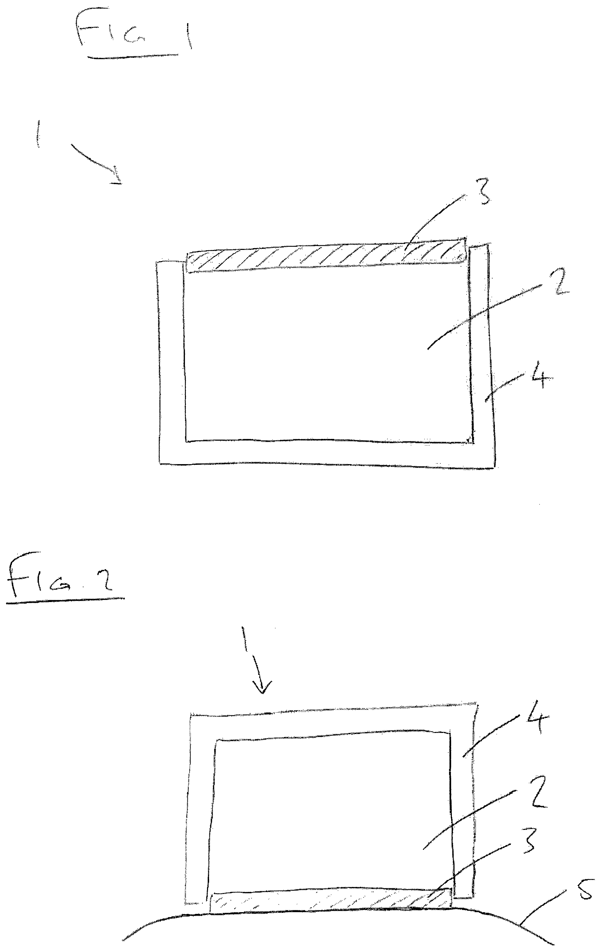

An electrode according to an embodiment is shown in figure 1. The electrode 1 comprises a reservoir 2 containing therein a liquid, such as an electrolyte. In fluid contact with the reservoir is an electrode material 3. The electrode material 3 is an electrical conductor and is the part of the electrode configured to be brought into contact with a subject (e.g. the skin of the subject) to measure electrical activity in the subject in the vicinity of the electrode. The electrode material 3 is a rigid (i.e. solid) material interfacing with the liquid in the reservoir 2. The electrode material 3 is configured to absorb the liquid in the reservoir 2.

Figure 2 shows the electrode of figure 1 in use, in which the electrode material 3 contacts the skin 5 of a subject. The part of the skin 5 of the subject to which the electrode 1 is applied may vary based on the type of measurement being carried out by the electrode. For instance, the electrode 1 may be placed on the scalp of a subject if the electrode 1 is to take EEG measurements, or the electrode 1 may be placed on the chest of a subject if the electrode 1 is to take ECG measurements. Performing measurements such as EEG, ECG or EMG on a subject may placing a plurality of the electrodes 1 in a desired spatial arrangement on the skin 5 of the subject. When in use, the electrode 1 is also configured to connect to a power source and a data processing means such as a computer or data logger. Said connections may be achieved by virtue of one or more cables connecting the electrode 1 to external parts such as a power source and a data processing means, and optionally to further ones of the electrode 1.

The constitution of the electrode material 3 and the liquid in the reservoir 2 are such that, due to the absorption of the liquid by the electrode material 3, the electrode 1 in use exhibits a low electrode-skin contact impedance over a long period of time. That is, the liquid in the reservoir 2 ensures continuous hydration of the electrode material which in turn provides a consistently low electrode-skin contact impedance. The reservoir of liquid and the absorptive electrode material enable the electrode to achieve low impedance to capture, for example, µV-scale fluctuations in electrical activity, and long-term stability in doing so.

The liquid in the reservoir 2 of the electrode is constituted to maintain an electrode-skin contact impedance of below 100 kΩ at a frequency of about 10 Hz, for a time period of at least 4 days. Preferably, the liquid in the reservoir 2 of the electrode 1 is constituted to maintain, when the electrode is in contact with the skin of a subject, an electrode-skin contact impedance of below 100 kΩ at a frequency of about 10 Hz, for a time period of at least 4 days in which the electrode 1 is continuously or periodically in contact with the skin 5 of the subject. This means that, over a period of 4 days, the electrode 1 is capable of exhibiting an electrode-skin contact impedance below 100 kΩ whenever the electrode material is brought into contact with the skin of the subject. That contact may occur continuously over that time period, in which case the electrode continuously maintains an electrode-skin contact impedance below 100 kΩ for the whole 4-day period. The contact may alternatively occur periodically (i.e. intermittently), in which case the electrode continuously maintains the electrode-skin contact impedance below 100 kΩ across the whole of each time interval in which the electrode is in contact with the skin (the electrode-skin contact impedance is undefined during the time intervals in which the electrode is not in contact with the skin). Additionally or alternatively, the liquid is constituted to maintain an electrode-skin contact impedance of below 100 kΩ at a frequency of about 10 Hz, at any point when the electrode comes into contact with the skin of a subject after provision of the electrode with the liquid in the reservoir and with the electrode material exposed. For instance, the electrode may comprise a seal (not depicted in the drawings) to cover the electrode material when not in use, and the liquid in the reservoir may be constituted such that any time in the 4-day time period after said seal is removed at which the electrode material contact skin of a subject, the electrode-skin contact impedance is below 100 kΩ.

As used herein, a time period "throughout which the electrode is periodically in contact with the skin of the subject" may refer to a time period spanned substantially across its whole width by intervals of electrode-skin contact. That is, a time period expressed in days throughout which the electrode is periodically in contact with the skin of the subject means a time period in which electrode-skin contact occurs both within the first day and the last day in that period. So, for instance, a time period of 4 days in which electrode-skin contact occurs of an interval on day 1 and an interval on day 2, but not at all on day 3 or day 4, is not considered a "time period of 4 days throughout which the electrode is periodically in contact with the skin of the subject" in the context of the present invention.

In general, it is desirable to maintain the electrode-skin contact impedance of the electrode during use as low as possible for as long as possible. Maintaining the electrode-skin contact impedance as low as possible ensures high-quality measurements with low noise, and maintaining the low impedance over as long a time period as possible ensure that said high-quality measurements with low noise are consistently achieved in long-term measurements (either continuous or periodic).

Therefore, the maintained electrode-skin contact impedance by the electrode 1 at about 10 Hz is below 100 kΩ, and may preferably be below 95 kΩ, more preferably below 90 kΩ, more preferably below 85 kΩ, more preferably below 80 kΩ, more preferably below 75 kΩ, more preferably below 70 kΩ, more preferably below 65 kΩ or most preferably below 60 kΩ.

Furthermore, the time period over which said electrode-skin contact impedance when the electrode is in contact with the skin is maintained, is at least 4 days, and may preferably be at least 5 days, more preferably at least 6 days, more preferably at least 7 days, more preferably at least 8 days, more preferably at least 9 days, more preferably at least 10 days, more preferably at least 11 days, more preferably at least 12 days, more preferably at least 13 days, more preferably at least 14 days, more preferably at least 15 days, more preferably at least 16 days, more preferably at least 17 days, more preferably at least 18 days, more preferably at least 19 days, more preferably at least 20 days, more preferably at least 21 days, more preferably at least 22 days, more preferably at least 23 days, more preferably at least 24 days, more preferably at least 25 days, more preferably at least 26 days, more preferably at least 27 days, more preferably at least 28 days, more preferably at least 29 days, and most preferably at least 30 days. As described herein, said time period may be a time period in which the electrode is continuously or periodically in contact with the skin of the subject.

The defined electrode-skin impedance is maintained by the electrode for the defined time period at a frequency (i.e. AC frequency) of about 10 Hz. The electrodes described herein are however useful in applications where the AC frequency is different from 10 Hz, such as anywhere between 1 Hz and 50 Hz.

The inventors have studied various materials from which to form each component of the electrode to best achieve the performances discussed herein.

With regard to the constitution of the electrode material 3, conducting polymers are a particularly favourable family of materials. Conducting polymers can have high electrical conductivity, high mechanical strength and flexibility, and biocompatibility. Conducting polymers can be absorptive, and in particular, the present inventors have realised that conducting polymers are particularly effective to absorb liquids of the sort that are contained in the reservoir 2 of the electrode 1 (such as deep eutectic solvents and ionic liquids). By forming the electrode material 3 such that it comprises an electrically conducting polymer, the quality of electrode readings in use therefore remains particularly high for particularly long periods of time because the liquid in the reservoir 2 can be effectively absorbed by electrode material 3 to continuously hydrate the electrode material 3, and the electrode remains robust and precise due to the strength and high electrical conductivity of the conducting polymer. The inventors have found PEDOT:PSS to be a particularly favourable conducting polymer for achieving these advantages. Preferably therefore, the electrode material 3 comprises PEDOT:PSS.

The electrode material 3 may also comprise additional components as well as the bulk material (e.g. PEDOT:PSS) from which it is formed. An additional component of the electrode material 3 may be conducting particles of metal, such as silver particles. The dispersion of such particles in the electrode material 3 may enhance the conductivity of the electrode and improve the precision of measurements. Another additional component of the electrode material 3 may be natural rubber, such as latex, which can enhance the mechanical strength and flexibility of the electrode 1 and thus the ability of the electrode 1 to adhere to the skin.

With regard to the constitution of the liquid in the reservoir 2, electrolytes are particularly favourable. The inventors have found that deep eutectic solvents and ionic liquids are beneficial when used as the liquid to be contained in the reservoir 2 of the electrode 1. Deep eutectic solvents and ionic liquids have useful physical properties including markedly low vapour pressures, making them much less susceptible to drying out over time than other types of liquids and gels and particularly those that are commonly used in conventional electrodes. Because of the low tendency of deep eutectic solvents and ionic liquids to dry out over time, when contained in the reservoir 2 of the electrode 1, these liquids may continue to be absorbed into the electrode material 3 and provide the beneficial low electrode-skin contact impedance for a long amount of time before depletion. In other words, providing a deep eutectic solvent or ionic liquid in the reservoir 2 of the electrode 1 enables effective continuous hydration of the electrode material 3 for especially long periods of time, thereby maintaining low electrode-skin contact impedance and high-quality readings for long periods of time. In terms of manufacturing the electrode 1, the low tendency of deep eutectic solvents and ionic liquids to evaporate is also useful, as discussed further below.

The inventors have found that deep eutectic solvents are especially suited to being used as the liquid contained in the reservoir 2 of the electrode 1. Deep eutectic solvents have an especially low tendency to dry out over time, particularly favourable viscosities and tendencies to be absorbed into electrode materials, and relatively low cost compared with ionic liquids. A particularly favourable liquid contained in the reservoir 2 of the electrode 1 is therefore a liquid that is or comprises a deep eutectic solvent.

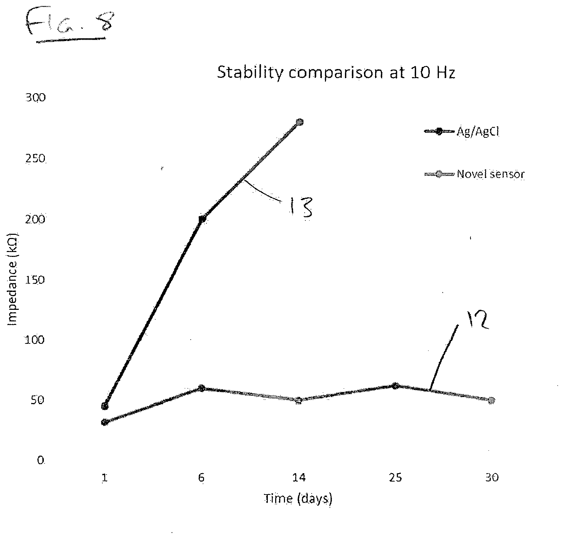

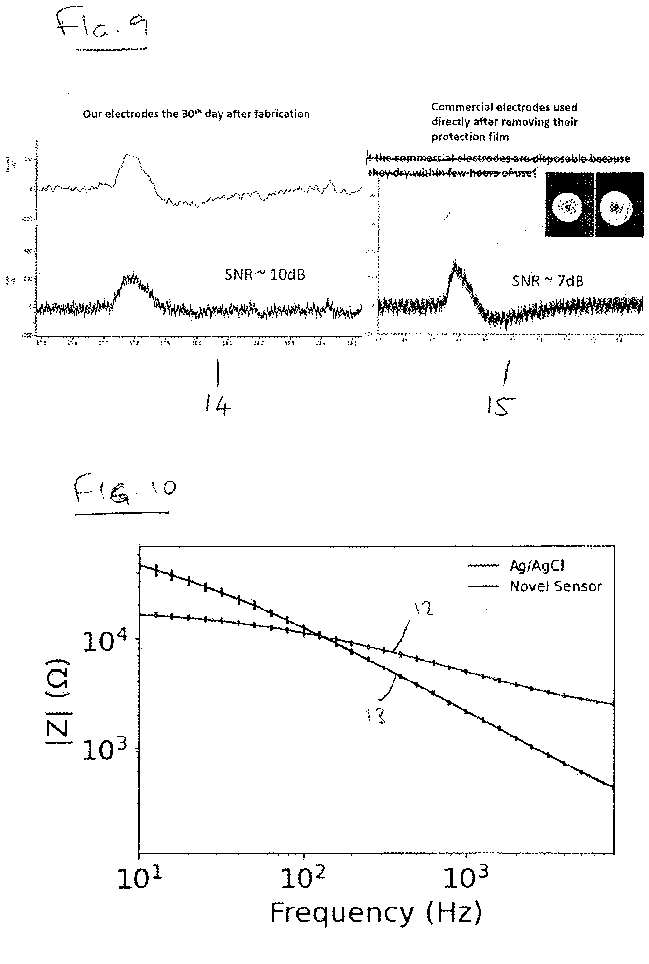

A preferable arrangement of the electrode 1 is one in which the electrode material 3 comprises a conducting polymer and the liquid in the reservoir 2 comprises a deep eutectic solvent. Most preferable is an arrangement in which the electrode material comprises PEDOT:PSS and the liquid in the reservoir 2 comprises a deep eutectic solvent. The inventors have found that, when a layer PEDOT:PSS is used as the electrode material 3 and is brought into continuous contact with a reservoir of deep eutectic solvent, the absorption of the deep eutectic solvent into the PEDOT:PSS layer and the minimal tendency of the deep eutectic solvent to dry out leads to continuous hydration of the PEDOT:PSS over a long period of time, and the ability of the electrode to exhibit very low contact impedances consistencies over these long periods of time. The performance of an electrode of this sort is displayed by figures 8 and 9.

Also shown in figure 1 and figure 2 is a housing 4 of the electrode 1. The housing 4 contains the reservoir 2, and is formed of a biocompatible polymer. When present, the housing 4 forms a solid structure around the reservoir such that the reservoir 2 is enclosed by the housing 4 and the electrode material 3. The housing may therefore enhance user convenience by providing a handle by which the user can place the electrode in the desired location on the skin 5 of a subject. The housing may have a simple structure as shown in figures 1 and 2 which only functions to enclose the reservoir in a casing. The housing may be formed by additive manufacturing. The housing may be a rigid structure or may have a degree of flexibility.

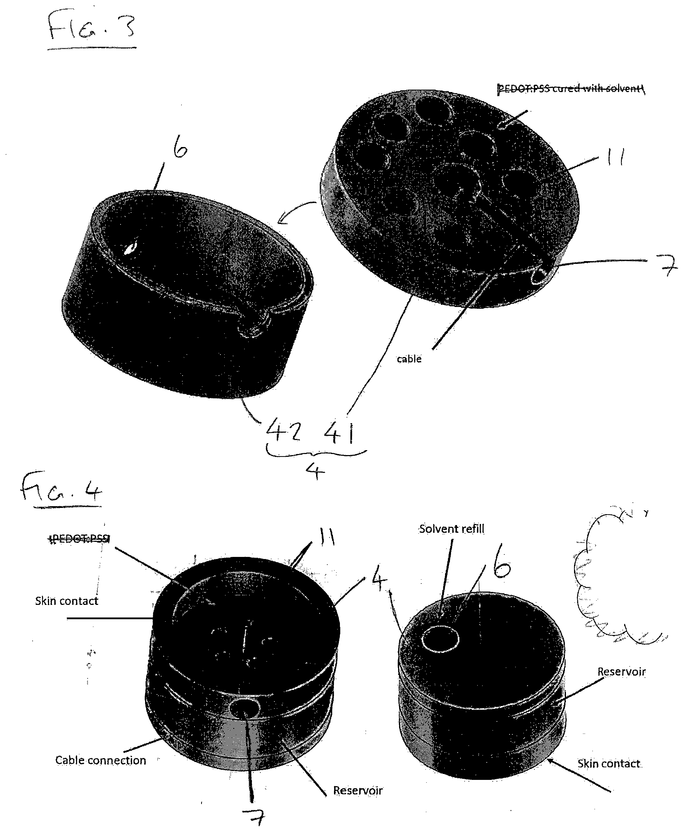

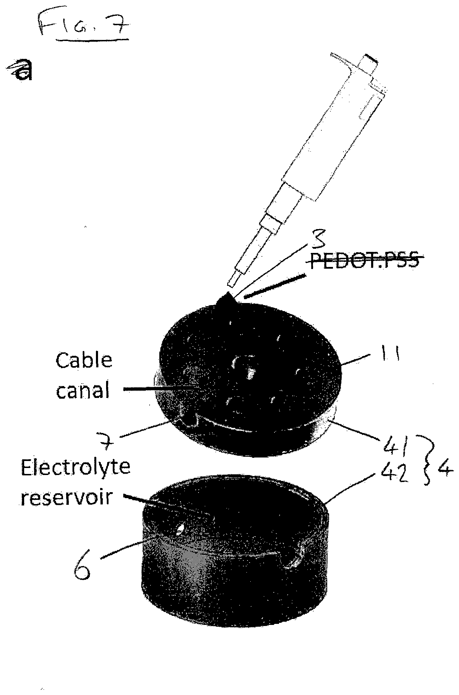

Figure 3 shows a preferred arrangement of the housing 4. In figure 3, the housing 4 is shown dismantled into two separate parts - inner part 41 and outer part 42. The inner part 41 and outer part 42 may be separate pieces to be attached together before use (e.g. by inserting the inner part 41 into the outer part 42), or the inner part 41 and outer part 42 may form a monolithic piece. When assembled together, a void is left in the outer part 42 below the inner part 41 to form a lower region of the reservoir for introduction of the liquid. The inner part 41 comprises one or more through-holes 11 providing a path of fluid communication between the aforementioned lower region of the reservoir and the location of the electrode material in the assembled electrode. The provision of through-holes 11 in the inner part 41 as shown in figure 3 may be useful in manufacture of an electrode, as discussed further below. Figure 7 shows a similar arrangement to that of figure 3, with a preferred arrangement of through-holes 1 1 in the inner part 41 of the housing 4.

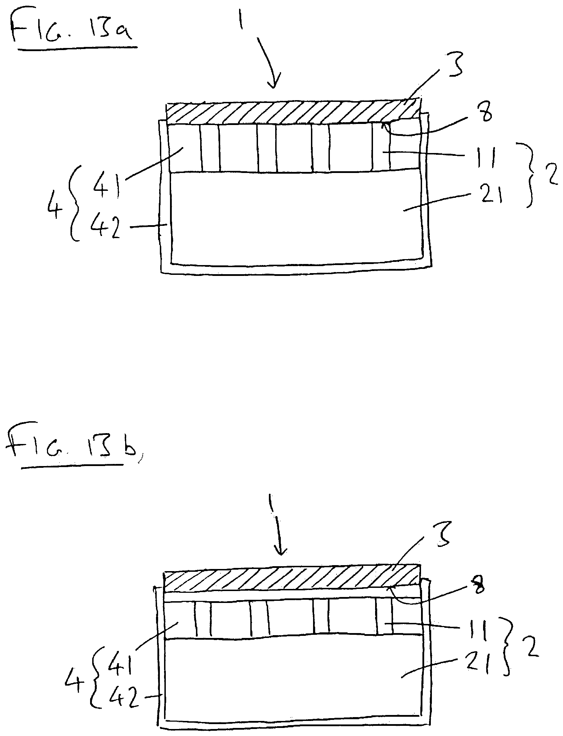

Figures 13a and 13b show in cross-section an assembled electrode 1 of a similar arrangement to that shown schematically in figures 3 and 7. The electrode 1 comprises housing 4 formed of inner part 41 and outer part 42. The housing 4 contains reservoir 2 which itself contains the liquid as described herein. The reservoir 2 comprises a reservoir lower region 21 which, in the orientation depicted in figures 13a and 13b, is below the housing inner part 41. Housing inner part 41 comprises one or more through-holes 11 that are in fluid communication with the lower region 21. Thus, in this arrangement, the reservoir 2 that contains the liquid comprises at least the following two distinct regions: the lower region 21; and the space within the one or more through-holes 11. Electrode material 3 is in contact with the reservoir 2 as described herein, and thereby configured to absorb the liquid from the reservoir 2. In the arrangement of figures 13a and 13b, the electrode material 3 is in contact with or in close proximity to openings ("upper openings") of the through-holes 11 on the side of the housing inner part 41 opposite to the lower region 21 of the reservoir 2. As shown in figure 13a, the layer of electrode material 3 in the assembled electrode may be in contact with both an upper surface of housing inner part 41 and the portion of the reservoir existing at the upper openings of through-holes 11, such that the electrode material 3 is configured to absorb liquid directly from through-holes 11. Additionally or alternatively, as shown in figure 13b, there may be a spatial separation between the upper openings of through-holes 11 and the layer of electrode material 3, forming a region (e.g. a narrow region) between the upper openings of through-holes 11 and the electrode material 3 into which the liquid may flow, which region thereby forms another part of the reservoir 2, and which region is itself in contact with electrode material 3. In this latter arrangement, the liquid effectively wets the upper surface of the inner part 41 of the housing 4, with the electrode material 3 placed on top of the wetted upper surface of inner part 41.

As used herein, the "upper openings" of through-holes 1 1 refers to the openings of through-holes 11 on the side of the through-holes opposite to the reservoir lower region 21, which are the top openings in the orientation depicted in figures 13a and 13b. Likewise, the "upper surface" of the housing inner part 41 refers to the surface of the housing inner part on which the through-hole upper openings exist.

The formation of housing 4 from an inner part 41 and outer part 42, as depicted in figures 3, 7 and 13, may provide advantageous structural rigidity to the assembled electrode 1, and provision of the through-holes 11 allows the liquid to favourably diffuse through the electrode material to provide the various advantages described herein. The provision of inner part 41 with through-holes 11 is also useful in the manufacture of the electrode 1, due to the supporting effect of the housing inner part 41 during deposition of electrode material 3, as described elsewhere herein.

As shown in figure 3, the housing 4 comprises a first opening 6 for introducing the liquid to the reservoir while the electrode material is in contact with a subject. That is, the first opening 6 is positioned towards the side of the housing opposite to the side that contacts the skin of the subject in use, and provides a passage from the outside to the reservoir. When the electrode is in use, eventual depletion of the liquid in the reservoir may cause the electrode-skin contact impedance to increase and the quality of measurements to decrease after some time. Further liquid may be introduced directly to the reservoir through opening 6, enabling the re-filling of the electrode with liquid without having to dismantle the electrode or remove it from the skin of the subject. After re-filling, the electrode material may begin to be continuously hydrated again, allowing the electrode to exhibit a low electrode-skin contact impedance and to produce high-quality results again. By provision of the first opening 6, the reservoir could in principle be re-filled an unlimited number of times, such that the volume capacity of the reservoir places no limit on the length of time over which the electrode may produce high-quality measurements due to having low electrode-skin contact impedance. When the housing 4 comprises inner part 41 and outer part 42 as described herein (shown in figures 13a and 13b), the first opening 6 may communicate specifically with the aforementioned lower region 21 of the reservoir 2.

As also shown in figure 3, the housing 4 comprises a second opening 7 to receive a cable. The second opening may provide a secure and robust means for housing a cable to connect the electrode to a power source and/or data logger or computer as discussed above.

Figure 4 shows another alternative arrangement of the housing 4, in which the first opening 6 for introducing liquid to the reservoir is placed on the flat surface of the housing 4 opposite to the side which comes into contact with the skin of the subject during use. The advantages of the first opening 6 and the second opening 7 are as discussed in relation to figure 3. The housing arrangement in figure 4 is shown in its fully constructed form rather than dismantled form.

The precise shape of the housing is not particularly limited. From a perspective of convenience of manufacture it may be favourable for the housing to be formed in a substantially cylindrical shape, as shown in figures 3, 4 and 7. However, the housing may also be formed in any other three-dimensional shape.

Optionally, a cable is attached to the electrode 1 so as to be in electrical contact with the electrode material 3 and form a biosensor for long-term, continuous or semi-continuous measurements in applications such as EEG, ECG or EMG.

The electrode 1 is manufactured by a method that comprises, at a minimum, steps of: introducing a liquid to a reservoir 2, the liquid in the reservoir defining a liquid surface; and depositing an electrode material 3 on the liquid surface. The liquid in the reservoir is constituted such that a surface tension at the liquid surface 8 supports thereon the electrode material 3.

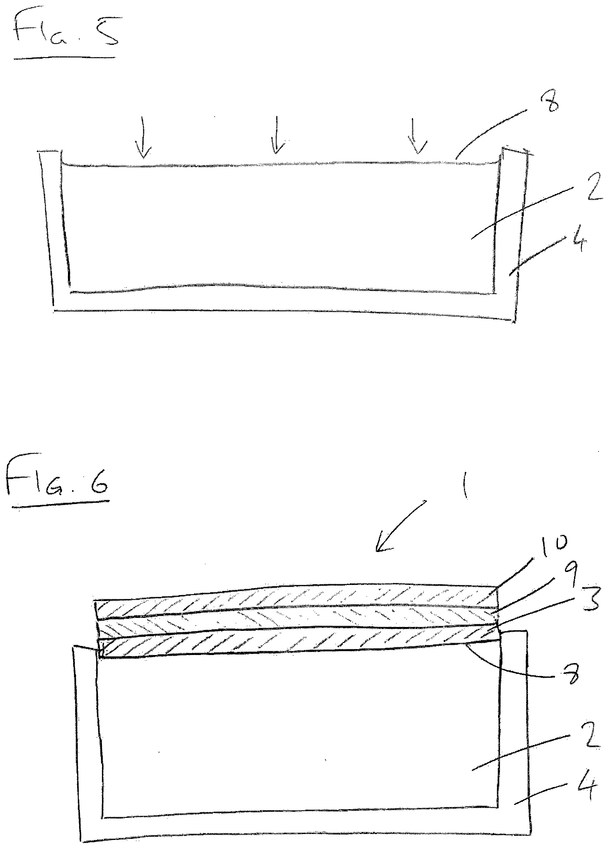

Figure 5 depicts a reservoir 2 of liquid, defining a liquid surface 8, on which an electrode material can be deposited to form the electrode of the present invention (e.g. the electrode 1 depicted in figure 1). The arrows in figure 5 indicate the deposition of electrode material onto liquid surface 8. The surface tension at the liquid surface 8 supports the electrode material 3 such that the electrode material does not sink or mix into the liquid in the reservoir. Instead, the electrode material 3 remains on top of the liquid surface 8 and forms a layer of the electrode material 3, which in the eventual finished electrode 1 is a solid absorbent layer of electrode material 3 configured to absorb the liquid on which it is deposited. Advantageously, the liquid surface 8 provides a flat, level and even surface for deposition of the electrode material 3. In this way, the electrode material 3 may be deposited uniformly and in a highly controlled way such that a flat and uniform electrode material layer may be formed with minimal imperfections in shape. Minimising imperfections in the shape of the eventual layer of electrode material is useful in ensuring accuracy and quality of measurements obtained from the electrode, with minimal noise.

In order so that a flat and uniform layer of electrode material 3 may be formed on top of the liquid surface 8, it is desirable that the electrode material 3 is deposited in fluid form onto the liquid surface. For instance, the electrode material 3 may be dissolved to form a solution of electrode material 3, which is deposited onto the liquid surface 8 and subsequently allowed to dry to form a solid electrode layer. As an example, when the electrode material 3 is or comprises a conductive polymer such as PEDOT:PSS, the conductive polymer may be deposited in solution, and subsequently dried so as to evaporate the solvent and leave a dry layer of electrode material 3 being or comprising the conductive polymer. When the electrode material 3 is deposited in solution, the solvent of the electrode material solution is not particularly limited and may be, for instance, water. Depositing the electrode material 3 in fluid form such as in solution enables the fluid electrode material 3 to settle in a flat and uniform layer on top of the liquid surface 8 before hardening, such that the eventual solid electrode material layer is advantageously flat and uniform. When the electrode material 3 is deposited in fluid form, it may be deposited by drop-casting.

As depicted in figure 5, the reservoir 2 may be formed within a housing 4, which may be the same housing 4 as discussed elsewhere herein. For instance, the housing 4 may take the form depicted in figure 3, 4, 7 or 13. Figure 7 is an image depicting the deposition, in liquid form, of electrode material onto an inner housing part 41, similar to that depicted in figure 3, which forms part of the overall housing 4. The housing 4 may be designed so as to facilitate the method of manufacturing the electrode. For instance, the first opening 6 as depicted in figures 3, 4 and 7 provides a passage through which the liquid may be introduced to the reservoir 2 in the method. Liquid may be forced (such as pumped) through the first opening 6, with the housing 4 held upright such that as the liquid enters in the reservoir 2 it settles in the reservoir 2 to form a substantially flat liquid surface 8 (as shown in figure 5). Whatever the exact shape of the housing 4, the housing 4 may preferably be fabricated from a biocompatible polymer by additive manufacturing, such as 3D printing. This technique can ensure precise tuning of the shape of the housing so as to optimise the subsequent introduction of liquid to the reservoir 2 in the housing, and the formation of the flat and uniform liquid surface 8 onto which electrode material 3 may be deposited.

The arrangement of housing 4 shown in figure 7, shown also in cross-section as part of a fully assembled electrode in figures 13a and 13b, are particularly suitable in the method of manufacture of the electrode 1. These forms of housing comprise housing inner part 41 with one or more through-holes 11, and housing outer part 42. The housing inner part 41 may comprise, for instance, seven through-holes 11 as depicted in figure 7, however the number of through-holes is not particularly limited. Liquid rises through through-holes 11 during introduction of liquid to the reservoir. The level of formation of the eventual liquid surface 8, for deposition thereon of electrode material 3, depends on the volume of liquid introduced to the reservoir 2 before the deposition of electrode material 3.

The quantity of liquid introduced is at a minimum sufficient to fill the lower region 21 of the reservoir below the housing inner part 41, and fill the space defined within the through-holes 11 in the housing inner part 41. If only this minimum quantity of liquid is introduced, then the eventual liquid surface 8 forms at the upper openings of through-holes 11, flush with the upper surface of the housing inner part 41. This is the arrangement depicted in figure 13a. The liquid surface 8 may then be formed of a number of individual liquid surfaces corresponding to the number of through-holes 11 in the housing inner part 41. When the liquid surface 8 forms at the level of the through-hole upper openings, electrode material 3 is subsequently deposited on a flat surface formed of the liquid surface 8 (which may be a plurality of liquid surfaces corresponding to a plurality of through-holes) and the upper surface of the housing inner part 41 that is flush with the aforementioned through-hole openings. The electrode material 3 is then supported by the housing inner part 41 as well as the surface tension of the liquid surface 8.

The quantity of liquid introduced to the reservoir 2 may instead be sufficient to form the liquid surface 8 above the level of the through-hole 11 upper openings, such that there is a spatial separation between the housing inner part 41 and the eventually formed layer of electrode material 3. That is, the introduced liquid may wet the upper surface of the housing inner part 41 for deposition of electrode material 3 onto that wetted surface. This effectively corresponds to a liquid surface 8 positioned at a level slightly above the level of the upper openings of through-holes 11, which is the arrangement shown in figure 13b. In this case, electrode material 3 rests on the liquid surface 8 separated by a distance (e.g. a short distance) from the upper surface of housing inner part 41, and the electrode material 3 is supported by the liquid surface tension. In this case, the housing inner part 41 still plays a substantial part in supporting the electrode material in place.

The position at which the eventual liquid surface 8 forms in relation to the through-hole openings is not particularly limited, nor is the degree of contact between the formed layer of electrode material 3 and the housing inner part 41, as long as the surface tension of the liquid surface 8 acts to support the electrode material 3 thereon to some degree during the method of manufacture of the electrode. Arrangements falling somewhere between that shown in figure 13a and figure 13b are also possible, for instance where the electrode material 3 is in contact with some parts of the upper surface of housing inner part 41, but is separated spatially from the housing close to the openings of the through-holes 11. In whatever arrangement, the surface tension of the liquid surface 8 advantageously prevents penetration of the electrode material 3 into through-holes 11 and the mixing of electrode material with the bulk liquid in the reservoir 2. The formation of an electrode layer 3 on or close to a housing inner part 41 may precede the formation of one or more subsequent electrode layers as described herein (for instance, with reference to figure 6.

When the electrode material 3 is deposited on the liquid surface in a form that requires subsequent drying to form a solid layer of electrode material 3 (e.g. deposited in solution), additional steps may be included in the method of manufacturing the electrode 1 in order to accelerate said drying. A step of curing the electrode material 3 on the liquid surface may efficiently produce a solid electrode layer by evaporating off any solvent present in the electrode material 3 in the form in which it is deposited. For instance, a solution in water of conducting polymer may be cured to evaporate all or most of the water therein, so as to leave a dry layer of the conducting polymer, configured then to absorb the liquid in the reservoir 2 with which it is in contact. Therefore, a preferable method of manufacture of an electrode 1 involves curing the electrode material 3 after it has been deposited in fluid form (e.g. in liquid solution) on top of the liquid surface 8 and supported thereon by the surface tension of the liquid in the reservoir. This method produces a highly uniform electrode, which also possesses the advantages described herein that result from the subsequent absorption by the electrode material 3 of the liquid in the reservoir 2.

It is desirable that, during the drying and hardening of the electrode material 3 after its deposition on the liquid surface 8, the liquid itself in the reservoir does not evaporate to any significant degree. That is, the method of manufacturing the electrode may require causing the evaporation of a solvent in which the electrode material 3 has been deposited on the liquid surface 8, while simultaneously preventing significant evaporation of the liquid in the reservoir 2. It is desirable to prevent significant evaporation of the liquid in the reservoir 2 because, if the liquid in the reservoir 2 evaporates significantly during the manufacture, the uniformity of the eventual layer of electrode material 3 may be compromised. For instance, evaporation of the liquid in the reservoir 2 could disrupt the uniformity of the liquid surface 8 on which the electrode material is supported. If the liquid in the reservoir 2 evaporates to a degree while the electrode material 3 is still soft or fluid, rather than completely rigid, the electrode material 3 may seep into holes or cavities in the reservoir 2 where liquid is no longer present before or while it is hardening and thereby the intended uniform (e.g. flat) shape of the electrode material layer may not be obtained. Evaporation of the liquid in the reservoir 2 could also cause the height of the liquid surface 8 to change after the deposition of the electrode material 3, so that the electrode layer does not form at the desired height. In the case where the liquid surface 8 is intended to be formed flush with upper openings of through-holes 11 (see figure 13a), evaporation of the liquid may cause electrode material to enter the through-holes 11 prior to hardening, resulting in an undesirably non-uniform shape of the eventual electrode material layer. These imperfections in the height and/or form of the electrode material layer caused by substantial evaporation of the liquid in the reservoir 2 during manufacture may in turn lead to reduced quality or accuracy of measurements produced by the electrode 1 when in use after manufacture.

It is especially important to design the electrode 1 and the manufacturing method that produces it so as to prevent substantial evaporation of the liquid in the reservoir 2 when, as discussed above, the method involves a heat treatment step such as curing to enable or accelerate the drying of the electrode material 3 to form a solid layer. In that case, constituting the liquid in the reservoir 2 from the same constituents as the solvent in which the electrode material 3 is dissolved, for instance, would inevitably result in substantial evaporation of the liquid from the reservoir 2. Instead, it is desirable for the liquid in the reservoir 2 that forms the liquid surface 8 to be constituted such that it does not evaporate significantly during the drying (e.g. curing) of the electrode material. This can be achieved by selection of an appropriate liquid to be introduced to the reservoir 2 in the initial step of the method. Therefore, preferably, the liquid in the reservoir 2 is constituted such that it does not evaporate significantly during drying (e.g. curing) of the electrode material.

The tendency of the liquid not to evaporate significantly from the reservoir 2 during drying (e.g. curing) of the electrode material 3 may defined in terms of vapour pressure of the liquid. For instance, the liquid introduced to the reservoir 2 may be constituted so as to have a vapour pressure of below 1 kPa, more preferably below 900 Pa, more preferably below 800 Pa, more preferably below 700 Pa, more preferably below 600 Pa, more preferably below 500 Pa, more preferably below 400 Pa, more preferably below 300 Pa, more preferably below 200 Pa, more preferably below 100 Pa, more preferably below 90 Pa, more preferably below 80 Pa, more preferably below 70 Pa, more preferably below 60 Pa, more preferably below 50 Pa, more preferably below 40 Pa, most preferably below 30 Pa., the vapour pressure being defined at a temperature of about 313 K. For comparison, the vapour pressure of water at this temperature is about 7.4 kPa, indicating a far higher tendency to evaporate.

As regards the liquid to be introduced to the reservoir 2 and on which to subsequently deposit electrode material, the inventors have determined that deep eutectic solvents and ionic liquids are particularly suitable due to their generally low tendencies to evaporate compared with other liquids. As discussed herein, the low vapour pressures of deep eutectic solvents and ionic liquids make them highly suitable as the liquid to be contained in the reservoir 2 of an electrode, because in use of the electrode 1 these liquids can continuously hydrate the electrode material 3 for a long time by being absorbed into it, thereby providing the electrode 1 with a low electrode-skin contact impedance over a long period of time. The inventors have determined that these types of liquids also present significant advantages during the manufacture of such electrodes. That is, the low tendency of deep eutectic solvents and ionic liquids to evaporate, such as during a step of curing the electrode material 3, improves the eventual uniformity of the electrode material layer and the precision with which it can be formed. Furthermore, deep eutectic solvents and ionic liquids have been found to form a liquid surface 8 with substantial surface tension which is particularly suitable for deposition thereon of electrode material. Deep eutectic solvents and ionic liquids therefore are multiply advantageous, in that they not only improve the performance of the electrode by continuously hydrating the electrode material 3 over a long time, but they also greatly facilitate the manufacture of a uniform and reliable electrode material layer.

As discussed herein, deep eutectic solvents are especially suitable as the liquid in the reservoir 2, because of their especially low tendency to evaporate, particularly favourable viscosities and tendencies to be absorbed into electrode materials, and relatively low cost compared with ionic liquids. Deep eutectic solvents also form a particularly strong liquid surface 8 with a surface tension able to stably support deposition of an electrode material such as a solution of PEDOT:PSS and to prevent penetration of said electrode material into the reservoir 2 of liquid. A particularly preferable method of manufacturing an electrode 1 thus comprises introducing a deep eutectic solvent to the reservoir 2, and subsequently depositing electrode material 3 (such as PEDOT:PSS in solution) onto the liquid surface 8 formed by the deep eutectic solvent. The resulting electrode 1 can have an advantageously uniform electrode material layer 3, and also provides the various advantages discussed herein associated with continuous absorption of the deep eutectic solvent.

After the electrode material 3 has been deposited on the liquid surface 8, it can be advantageous to deposit, on top of the deposited electrode material 3, an additional amount of the liquid (i.e. the same liquid as is introduced to the reservoir at the start of the method). The additional amount of the liquid can be deposited by drop-casting to enable even dispersal. The electrode material 3 may be configured to absorb the liquid that is contained in the reservoir, thereby providing the advantages discussed herein of reduced electrode-skin impedance over long periods of time. In that case, depositing an additional quantity of the liquid on top of the electrode material 3 may enhance the speed with which the whole layer of electrode material 3 absorbs the liquid. That is, liquid may be absorbed into the electrode material 3 from both the surface facing the reservoir 2 (i.e. absorption of the liquid already in the reservoir 2), and the surface facing outwards from the reservoir (i.e. absorption of the additional liquid deposited on the electrode material 3). This may advantageously improve the uniformity with which the electrode material 3 is saturated with the liquid. In the case where the electrode material 3 is deposited on the liquid surface in solution, and subsequently dried (e.g. by curing), depositing an additional amount of the liquid on the electrode material 3 may also fill the space left by the evaporated solvent in which the electrode material 3 was initially dissolved. For example, when an aqueous solution of the electrode material PEDOT:PSS has been deposited on the liquid surface 8, and subsequently cured to dry the PEDOT:PSS, the additional deposited liquid may replace the water that has evaporated. This can prevent the resulting electrode 1 from having holes or cavities in the layer of electrode material 3 which could otherwise detriment the quality of measurements produced by the electrode 1. For the reasons discussed herein, the liquid in the reservoir 2 and deposited additionally on the electrode material 3 is preferably a deep eutectic solvent or an ionic liquid, most preferably a deep eutectic solvent.

The deposition of additional liquid on top of the electrode material 3 may occur after curing of the electrode material 3. The deposition of additional liquid on top of the electrode material 3 preferably occurs during curing of the electrode material. The deposition of additional liquid on top of the electrode material 3 could occur both during and after curing of the electrode material 3. Depositing at least some of the additional amount of the liquid on the electrode material 3 during the curing of the electrode material can reduce the overall time of manufacture of the electrode 1. Also, if the electrode material 3 was deposited on the liquid surface 8 in solution, then by depositing additional liquid on the electrode material 3 while it cures, the solvent that evaporates during the curing may be immediately replaced by the additional liquid. This avoids there being a substantial period of time in which an empty void is left by the evaporated solvent. Stability of the produced electrode 1 may therefore be enhanced.

When the method includes deposition of additional liquid on the electrode material 3, the method may further comprise a subsequent, additional curing step in which the electrode material 3 with the added additional liquid is cured.

The electrode 1 shown in figure 1 , which is in accordance with an embodiment and is also manufactured by a method in accordance with other embodiments, is depicted with a single layer of electrode material 3. However, the electrode 1 is not limited to having only a single layer of electrode material 3, nor is the method of manufacturing said electrode limited to only producing a single layer of electrode material 3.

When the electrode material 3 deposited on the liquid surface 8 has dried and hardened, it may be termed a "first electrode layer". It can be advantageous to provide, on top of the first electrode layer, on or more further layers of the electrode material. Figure 6 depicts an electrode 1 having a plurality of electrode layers. Three electrode layers are shown in figure 6: the first electrode layer 3 as present in the electrode 1 depicted in figure 1; a second electrode layer 9, and a third electrode layer 10. An electrode 1 may in principle consist of any number of distinct electrode layers, including two electrode layers, or more than three electrode layers. A second electrode layer, which is distinct from the first electrode layer, can be formed by depositing in fluid form (such as in solution) further electrode material on top of the already dried electrode material that forms the first electrode layer, and subsequently drying the newly deposited electrode material to form the second electrode layer. The drying to form the second electrode layer may be achieved by curing, which may occur subsequent to a curing step that facilitated or enabled drying of the first electrode layer 3. Further electrode layers, such as the third electrode layer 10 and any number of subsequent electrode layers, can be formed similarly by depositing and drying (e.g. curing) further electrode material on top of the preceding already-dried layer. For example, forming an electrode 1 having six electrode layers in total has been found beneficial in terms of maintenance of a low electrode-skin contact impedance for a long period of time.

A preferable method of manufacturing an electrode 1 having a plurality of electrode layers may therefore comprise: forming the first electrode layer by introduction of liquid to the reservoir 2, deposition of electrode material 3 on the liquid surface 8 formed by said liquid, and subsequent drying (e.g. curing) of the electrode material; and forming a second electrode layer 9 by depositing and drying (e.g. curing) an additional amount of electrode material on the first electrode layer. Optionally, one or more further electrode layers are formed by sequentially depositing and curing an additional amount of the electrode material on the preceding electrode layer (e.g. third electrode layer 10 may be formed on the second electrode layer 9).

In the case where a plurality of electrode layers are formed in the electrode 1, any of the features or method steps that have been described herein in relation to forming the initial layer of electrode material may also be employed in formation of the subsequent layer(s). For instance, after formation of the plurality of electrode layers, an additional amount of the same liquid that is present in the reservoir may be deposited onto the electrode material forming the layers. The quantity of liquid forming that additional amount is not particularly limited. For instance, after formation of the plurality of electrode layers, the additional amount of the liquid may be deposited onto the top layer in a quantity that is determined based on a proportional relationship with the number of layers (e.g. an electrode having four electrode layers may be subject to twice as much additional liquid deposition onto the top layer as an electrode having two electrode layers). Additionally or alternatively, each layer of electrode material that is deposited to form the electrode may have deposited thereon an additional quantity of the same liquid that is present in the reservoir. As an example, during or after curing of the electrode material deposited on top of the already-formed first electrode layer 3 in order to form the second electrode layer 9, an additional quantity of the liquid that is contained in the reservoir 2 may be deposited (e.g. drop-casted) on top of the electrode material forming the second electrode layer 9. This may provide similar advantages as described above in relation to the first electrode layer 3, in particular, the filling of voids left by evaporated solvents to enhance stability of the second electrode layer 9. Additional liquid may be deposited on every electrode layer, on none of the electrode layers, or on some of the electrode layers. Furthermore, for each electrode layer that has additional liquid deposited thereon to enhance stability, additional curing steps may be employed as described herein in relation to the first electrode layer.

The performance of an electrode 1 produced by the method described herein may be further improved by including a treatment step involving immersing the one or more layers of electrode material in an immersion liquid, which is preferably water. Other immersion liquids that may be suitable are deep eutectic solvents or ionic liquids (e.g. the same constitution as the liquid that is in the reservoir 2), and various other solutions. The immersion step may take place over a time period of at least 12 hours, or more preferably at least 24 hours. The inventors have determined that immersing the electrode material in an immersion liquid such as water, in particular after curing of the electrode material, can result in enhanced long-term quality of measurements produced by the electrode 1. Immersion in the immersion liquid helps the electrode material to release excess of PSS (polystyrene sulfonate) which is insulating and acidic. This improves conductivity and biocompatibility of the electrode. When multiple layers of electrode material are formed, as discussed herein and as shown in figure 6, individual steps of immersion in water may occur after individual formation of certain layers or of every layer, or a single immersion step may occur after all layers have been formed. Preferably, after the electrode layers of only the electrode material are added and cured, the immersion step follows, and after the immersion, additional liquid (that is, the "liquid" that is present in the reservoir) is added and cured on top of the electrode layers.

An exemplary process for forming an electrode is as follows: (1) drop casting the electrode material to form a layer every 20-30 min and curing for an hour after the last layer is deposited; (2) immersion of the cured layers in water for 24 hours; (3) additional drop casting of the same liquid as that contained in the reservoir (e.g. 100 µl every 10-20 min) and curing for a time period that is proportional to the number of layers (e.g. around 50 µl for every layer so after formation of 6 layers of electrode material, 100µl of liquid is deposited three times, separated by 20 minutes, and the product is cured for 4 hours).

As described herein, various advantages are provided by the electrode 1 and its manufacturing method in accordance with various embodiments of the invention. An aspect of the invention may be regarded as the process that occurs within the electrode during use, that is, a process of continuous hydration of an electrode. As described herein, said continuous hydration occurs by bringing an absorbent electrode material into contact with a liquid, the liquid being constituted to maintain, when the electrode is in contact with the skin of a subject, an electrode-skin contact impedance of below 100 kΩ at a frequency of about (e.g. at) 10 Hz, for a time period of at least 4 days (e.g. throughout which the electrode is continuously or periodically in contact with the subject). This method of continuously hydrating an electrode may incorporate any one or any combination of features discussed herein in relation to other embodiments, such as embodiments of the electrode 1 itself or embodiments of the method of manufacturing said electrode 1.

### Experimental data

Figure 8 depicts a graph of how electrode-skin contact impedance at 10 Hz changes over time in respect of an electrode 1 of the present invention comprising a single layer of PEDOT:PSS as the electrode material 3 and a deep eutectic solvent as the liquid in the reservoir 2 (numeral 12), compared with a commercially available gel-assisted Ag/AgCl electrode (numeral 13), having the same skin contact area (that is, the same diameter and shape of electrode material). To obtain the results shown in figure 8, the electrode 1 of the invention was brought into contact with the skin of a subject separately for intervals on day 1, day 6, day 14, day 25 and day 30 of a total 30-day period (i.e. a time period of 30 days throughout which the electrode was periodically in contact with the skin of the subject). The commercially available Ag/AgCl electrode was brought into contact with the skin of a subject for intervals on day 1, day 6 and day 14. As shown in figure 8, the electrode 1 of the invention comprising PEDOT:PSS and deep eutectic solvent provides a substantial improvement in stability compared with the commercial electrode. The electrode 1 of the invention over the first 14 days shows a significantly smaller deviation in impedance than the commercial Ag/AgCl electrode, whose impedance increases by more than 5 times over that period. The electrode 1 of the invention also continues to show minimal increase in impedance over the remainder of the 30-day period. It can be seen that the electrodes of the invention maintain a low impedance for 30 days after fabrication while the gel-assisted electrodes impedance is rapidly increasing after being in open air for more than one day.

Figure 9 depicts EEG signals taken by the same electrode 1 of the invention 30 days after fabrication (numeral 14) and by the same commercial gel-assisted Ag/AgCl electrode directly after removing its protective film (numeral 15). As shown in figure 9, even after 30 days from fabrication, the electrode 1 of the present invention produces signals of higher quality and lower noise than those produced by the fresh commercial electrodes. The signal-to-noise ratio (SNR) observed for the electrode of the invention was about 10 dB, whereas the SNR observed for the commercial electrode was about 7 dB.

Figure 10 depicts, over a range of frequencies, the electrode-skin impedance of the same electrode 1 of the invention (numeral 12), compared with that of the same commercial gel-assisted Ag/AgCl electrode (numeral 13), directly after fabrication. As described herein, the electrodes and methods of the present invention are of particular use in applications where the AC frequency is between 1 and 50 Hz. Figure 10 shows that, within this frequency range, even straight after fabrication the electrodes of the present invention outperform the commercial electrodes in terms of having a low electrode-skin impedance in use.

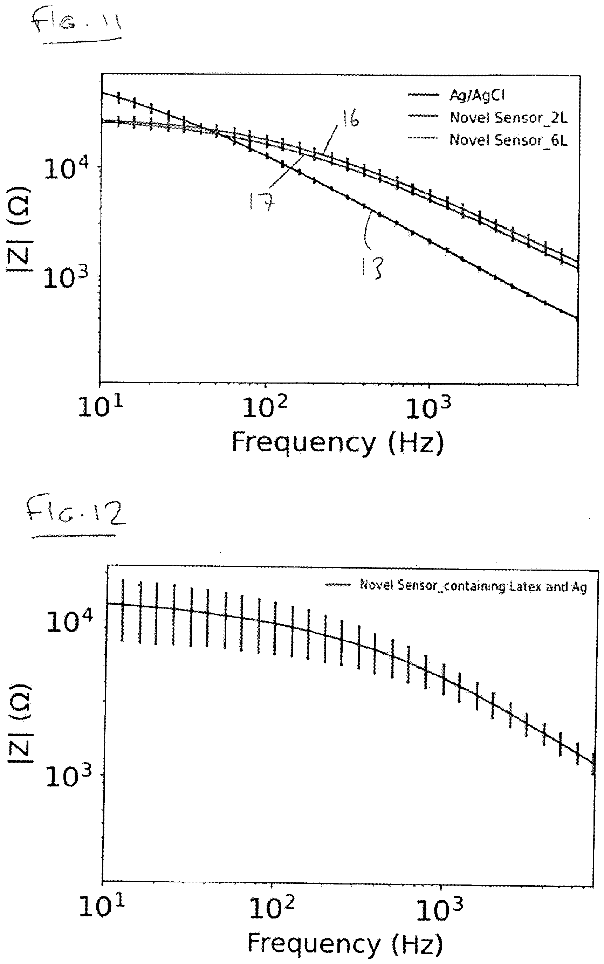

Figure 1 1 depicts, over a range of frequencies, the electrode-skin impedance of electrodes 1 of the invention having pluralities of electrode layers (numeral 16 = 2 layers; numeral 17 = 6 layers), compared with that of the same commercial gel-assisted Ag/AgCl electrode (numeral 13), directly after fabrication. In the 2-layer electrode and 6-layer electrode of the invention in this experiment, each layer of electrode material comprised 100 µl of PEDOT:PSS, and each electrode of the invention was also subjected to immersion in water for over 24 hours after fabrication. As shown in figure 11, the multi-layered electrodes of the invention also outperform commercial electrodes in the frequency range of interest (1-50 Hz) in terms of having a low electrode-skin impedance in use.

Figure 12 depicts, over a range of frequencies, the electrode-skin impedance of an electrode 1 of the invention, having six electrode layers of PEDOT:PSS (each layer comprising 100 µl of electrode material) and a deep eutectic solvent reservoir, and having both silver microparticles and latex added to the PEDOT:PSS electrode material. As shown in figure 12, the additives lowered the impedance to around 10 kΩ in the frequency range of interest (1-50 Hz).

The devices and methods described herein are suitable for EEG, ECG, EMG and similar techniques. However, devices having substantially the same arrangement may also be configured for use as electrodes in various other applications, as will be apparent to those skilled in the art. Any combination of the features and method steps described herein may be made without departing from the scope of the invention.

## Claims

1. An electrode comprising:
a reservoir containing a liquid; and
an electrode material in contact with the reservoir and configured to absorb the liquid,
wherein the liquid in the reservoir is constituted to maintain an electrode-skin contact impedance of below 100 kΩ at a frequency of about 10 Hz, for a time period of at least 4 days.

2. The electrode of claim 1, wherein:
the liquid in the reservoir is constituted to maintain an electrode-skin contact impedance of below 100 kΩ at a frequency of about 10 Hz, for a time period of at least 5 days, preferably at least 10 days, preferably at least 20 days, preferably at least 30 days; and/or
the liquid in the reservoir is constituted to maintain an electrode-skin contact impedance, at a frequency of about 10 Hz, of below 90 kΩ, preferably below 80 kΩ, preferably below 70 kΩ, preferably below 60 kΩ, over a time period of at least 4 days.

3. The electrode of claim 1 or claim 2, wherein the electrode material comprises an electrically conducting polymer, preferably wherein the electrode material comprises PEDOT:PSS, and further preferably wherein:
the electrode material additionally comprises conducting metal particles such as silver particles; and/or
the electrode material additionally comprises a natural rubber such as latex.

4. The electrode of any one of the preceding claims, wherein the liquid comprises a deep eutectic solvent or an ionic liquid, preferably wherein the liquid comprises the deep eutectic solvent.

5. The electrode of any one of the preceding claims, further comprising a housing containing the reservoir, the housing being formed of a biocompatible polymer, preferably wherein the housing comprises at least one of:
an opening for introducing the liquid to the reservoir while the electrode material is in contact with a subject; and
an opening to receive a cable.

6. A biosensor comprising:
the electrode of any preceding claim; and
a cable in electrical contact with the electrode material.

7. A method of manufacturing an electrode, the method comprising:
introducing a liquid to a reservoir, the liquid in the reservoir defining a liquid surface; and
depositing an electrode material on the liquid surface,
wherein the liquid is constituted such that a surface tension of the liquid supports the electrode material thereon.

8. The method of claim 7, further comprising:
curing the electrode material on the liquid surface to form a first electrode layer,
preferably wherein the liquid is constituted such that it does not evaporate significantly during the curing of the electrode material; and/or
at a temperature of about 313 K, the liquid has a vapour pressure of below 1 kPa, more preferably below 500 Pa, more preferably below 200 Pa, more preferably below 100 Pa, more preferably below 50 Pa, more preferably below 30 Pa.

9. The method of claim 7 or claim 8, further comprising:
depositing, on the electrode material, an additional amount of the liquid,
preferably wherein the additional amount of the liquid is deposited by drop-casting.

10. The method of claim 9, wherein:
the additional amount of the liquid is deposited on the electrode material during curing of the electrode material; and/or
the method comprises curing the additional amount of the liquid deposited on the electrode material.

11. The method of claim 8, comprising forming a plurality of electrode layers including the first electrode layer and a second electrode layer, wherein:
the second electrode layer is formed by depositing and curing an additional amount of electrode material on the first electrode layer; and
optionally, one or more further electrode layers are formed by sequentially depositing and curing an additional amount of the electrode material on the preceding electrode layer.

12. The method of claim 11, further comprising depositing an additional amount of the liquid on the electrode material forming the electrode layers, said additional amount being proportional to the number of electrode layers, preferably wherein:
the additional amount of the liquid is deposited while said electrode material is being cured; and/or
the additional amount of the liquid is cured after deposition on the electrode material.

13. The method of any of claims 8 to 12, further comprising immersing the cured electrode material in an immersion liquid for at least 12 hours, preferably wherein the immersion liquid is water.

14. The method of any of claims 7 to 13, comprising forming a housing by additive manufacturing, the housing containing the reservoir, preferably wherein the housing is formed of a biocompatible polymer.

15. A method of continuously hydrating an electrode, comprising:
bringing an absorbent electrode material of the electrode into continuous contact with a liquid,
wherein the liquid in the reservoir is constituted to maintain an electrode-skin contact impedance of below 100 kΩ at a frequency of about 10 Hz, for a time period of at least 4 days.
